# EUROPEAN PATENT SPECIFICATION

(11) **EP 4 117 450 B1**
(45) Date of publication and mention of the grant of the patent: **26.08.2026**
(21) Application number: 21710005.6
(22) Date of filing: 10.03.2021
(51) Int. Cl.: A23K 20/158, A23K 50/30

(54) **COMPOSITION FOR USE IN THE PROPHYLAXIS AND TREATMENT OF VIRAL INFECTIONS OF THE ASFARVIRIDAE FAMILY**
ZUSAMMENSETZUNG ZUR VERWENDUNG BEI DER PROPHYLAXE UND BEHANDLUNG VON VIRUSINFEKTIONEN DER FAMILIE ASPARVIRIDAE
COMPOSITION DESTINÉE À ÊTRE UTILISÉE DANS LA PROPHYLAXIE ET LE TRAITEMENT D'INFECTIONS VIRALES DE LA FAMILLE DES ASFARVIRIDAE

(30) Priority: 10.03.2020 BE 202005165
(43) Date of publication of application: 18.01.2023
(73) Proprietor: Nutrition Sciences N.V., 9031 Drongen (BE)
(72) Inventor: BRUGGEMAN, Geert, 8200 Brugge (BE); LANNOO, Kobe, 9031 Drongen (BE); ROLAND, Brugger, 9000 Gent (BE)
(74) Representative: Brantsandpatents bv
(86) International application number: PCT/EP2021/056046
(87) International publication number: WO 2021/180786

(56) References cited:
- WO-A1-2009/150281
- WO-A1-2019/169256
- CN-A- 106 234 770
- US-A1- 2016 250 239

## Description

### TECHNICAL FIELD

This invention relates generally to the field of methods for the prophylaxis and treatment of viral infections by the *Asfarviridae* family, such as African Swine Fever Virus (ASFV).

### BACKGROUND

In the recent years, outbreaks of several viruses have challenged the agricultural industry such as the swine industry worldwide. In 2015 the spread of Porcine Epidemic Diarrhea (PED) shocked the industry, now African Swine Fever (ASF) is changing the global pig production landscape. African Swine Fever Virus (ASFV) is a contagious, rapidly spreading, transboundary animal disease and a major threat to pork production globally. Handling these kind of threats needs a very broad approach including biosecurity measurements, veterinary precautions, nutritional adaptations and feed safety measurements to stop the disease from spreading.

Learnings from previous foreign animal disease outbreaks have shifted the focus from diagnosis and treatment to prevention and control. Keeping pathogens at bay is the first step to an effective on-farm biosecurity program. An often-overlooked aspect in biosecurity planning, however, is the role of feed and feed ingredients. In particular, it is known that feed may carry pathogens that are detrimental to animal health and welfare. As such, spreading of viral particles via the feed is an example of growing concern. To this purpose antiseptic products such as formaldehyde are commonly used to disinfect the feed.

WO 2019 169 256 describes chemical mitigants to combat African swine fever virus or classical swine fever virus. WO 2009 150 281 and CN 106 234 770 discuss the use of conjugated fatty acids for combatting viral infections in animals.

Despite these proposed solutions, the industry to date lacks adequate methods to prevent the spreading of viral particles and consequently to prevent viral infections caused by the *Asfarviridae* family. The present invention aims to provide a solution for the treatment and prevention of viral infections by the *Asfarviridae* family, such as African Swine Fever.

### SUMMARY OF THE INVENTION

The invention thereto aims to provide a composition for use in the treatment, suppression and/or prevention of African Swine Fever (ASF) according to claim 1. More in particular, the invention provides a composition comprising one or more medium-chain fatty acids (MCFAs) and/or derivatives thereof. Preferred embodiments are shown in claims 2 to 8.

A further aspect of the invention relates to a method of inhibiting viruses of the Asfaraviridae in animal feed or drinking water according to claim 9.

Another aspect of the invention relates to a method of lowering the viral titer of African Swine Fever virus in an animal feed or drinking water according to claim 12.

### DESCRIPTION OF FIGURES

**Fig. 1** shows a graph relating to data in support of the susceptibility of African Swine Fever Virus (ASFV) to medium chain fatty acids (MCFAs) in animal feed.
**Fig. 2** shows a graph relating to data in support of the susceptibility of African Swine Fever Virus (ASFV) to medium chain fatty acids (MCFAs) in animal drinking water.

### DETAILED DESCRIPTION OF THE INVENTION

The present invention concerns a composition such as a feed additive for use in the prevention, suppression and/or treatment of *Asfarviridae,* preferably African Swine Fever. In particular, said composition comprises one or more medium-chain fatty acids (MCFAs) and/or derivatives thereof. Throughout the present disclosure, especially in the examples, compositions or methods comprising fatty acids having a longest continuous carbon chain shorter than 5 or longer than 12 carbon atoms, compositions containing less than 40 % C8, and/or MCFAs administered at dosages below 1500 ppm or above 5000 ppm are not according to the invention and are included for comparative or illustrative purposes only.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

The term "medium-chain fatty acid" or "MCFA" as used herein, refers to fatty acids with a medium-chain length, wherein the fatty acids may be saturated or unsaturated. In the present invention, the longest continuous chain of an MCFA can consist of 5 to 12 carbon atoms, for example, valeric acid (C5), caproic acid (C6), caprylic acid (C8), pelargonic acid (9), capric acid (C10) or lauric acid (C12). The term MCFA also refers to medium-chain fatty acids that are chemically modified, and to medium-chain fatty acids that are provided with side-chains, such as, without limitation, one or more alkyl groups, preferably C1-C10 alkyl groups. As described herein, the term "medium-chain fatty acid (MCFA) derivative" refers to a fatty acid chain of which the carboxyl group is reversibly converted to a different group, preferably, but without limitation, to an amide, salt, ester or glyceride such as, for example, a mono-, di- or tri-glyceride.

For the purpose of the current invention, the term *"Asfarviridae"* refers to a family of double stranded DNA viruses , that mainly infect insects and mammalians such as pigs.

The term "African Swine Fever" as used herein, refers to an infectious disease caused by the African Swine Fever Virus (ASFV), which is a DNA virus belonging to the *Asfarviridae* family. At least 22 types exist which enables outbreaks to be traced to source. It is extremely resistant to putrefaction and sunlight, and can persist in refrigerated meat and carcasses for up to 6 months and for much longer when frozen. Infection is spread from pig to pig usually by aerosol from infected discharges and feces, by the bites of soft ticks, the bites of lice and flies and by direct inoculation from contaminated syringes. Infection can also be spread on contaminated implements and during transport. The virus can survive in carrier pigs, in infected buildings and for up to 4 years in infected *Ornithodorus* ticks. African Swine Fever is highly contagious and infection spreads rapidly through a unit, with clinical signs of fever beginning 4-5 days after infection and causing fever followed by dullness, breathing difficulty, vomiting, coughing, nasal and ocular discharge, abortion in pregnant sows, cyanosis of the extremities and death within 7 days.

The term "Feed Conversion Ratio" or "FCR" describes the efficiency with which the animals convert feed into the desired output. For animals raised for meat (such as beef cows, pigs, chickens and fish) the output is the flesh, that is, the body mass gained by the animal, represented either in the final mass of the animal or the mass of the dressed output.

The term "antiviral *in vitro* assay" as used herein refers to any method suitable to determine potential efficacy of an antiviral substance, compound or composition towards a chosen virus or viral family. Known methods comprise TCID₅₀ assays, EC₅₀/CC₅₀ assays, plaque assays, HAI (hemagglutination inhibition) assays and ELISA/Luminex.

Cytopathic effect or cytopathogenic effect (abbreviated CPE) is to be understood as structural changes in host cells that are caused by viral invasion. The infecting virus causes lysis of the host cell or when the cell dies without lysis due to an inability to reproduce. Both of these effects occur due to CPEs. If a virus causes these morphological changes in the host cell, it is said to be cytopathogenic. Common examples of CPE include rounding of the infected cell, fusion with adjacent cells to form syncytia, and the appearance of nuclear or cytoplasmic inclusion bodies. CPEs and other changes in cell morphology are only a few of the many effects by cytocidal viruses. When a cytocidal virus infects a permissive cell, the viruses kill the host cell through changes in cell morphology, in cell physiology, and the biosynthetic events that follow. These changes are necessary for efficient virus replication but at the expense of the host cell.

In a first aspect, the invention provides a composition for use in the prevention, suppression and/or treatment of *Asfarviridae,* preferably African Swine Fever, wherein said composition comprises one and/or more medium-chain fatty acids (MCFAs) or derivatives thereof. MCFA(s) are fatty acids with a medium-chain length, wherein the fatty acids may be saturated or unsaturated. According to the present invention, the longest continuous chain of an MCFA consists of 5 to 12 carbon atoms (C5 to C12). The use of MCFA as antibacterial agent has been extensively described. In addition, its use against viral infections has been documented as well. In pigs, MCFA was discussed to have activity against viral pathogens such as Porcine Epidemic Diarrhea virus (PEDv), Porcine Reproductive and Respiratory Syndrome virus (PRRSv) and Seneca Valley virus (SVA).

Preferably, the composition of the invention comprises one or more MCFAs chosen from C5 to C12 or derivatives thereof. The term MCFA refers to the free fatty acid form of the MCFA. In addition, derivatives of MCFA can also be used, wherein said derivatives of MCFA are medium-chain fatty acids that are chemically modified, and to medium-chain fatty acids that are provided with side-chains, such as, without limitation, one or more alkyl groups, preferably C1-C10 alkyl groups. MCFA derivatives include MCFAs of which the carboxyl group is reversibly converted to a different group such as but not limited to an amide, a salt, an ester or a glyceride such as, for example, a mono-, di- or tri-glyceride. In a preferred embodiment, said MCFAs used are either the free form or the salt form, such as the sodium, potassium or calcium salt of MCFA.

*Asfarviridae,* in particular African Swine Fever is a major health challenge in the animal husbandry sector, especially the swine sector, affecting both productivity and profitability, resulting in a huge economic impact of this disease on the sector. The inventors have found that a composition comprising at least one MCFA or MCFA derivative is surprisingly effective for the prevention, suppression, and/or treatment of this disease, thus allowing to significantly increase both the productivity and profitability.

Furthermore, African Swine Fever is a severe disease whose impact on animal well-being should not be underestimated. Unfortunately, infection leads in many cases to the death of the animal. The current invention thus provides a composition that can make a considerable contribution to the reduction of animal suffering and the improvement of animal welfare.

To date, no real solutions exist in order to prevent or stop African Swine Fever disease outbreaks. No vaccine is available yet. The current invention provides an efficient solution for the treatment and to prevention of *Asfarviridae,* in particular ASFV.

The inventors have found that supplying specific MCFA in the range of C5-C12, their derivatives and / or mixtures thereof to feed of animals my prevent and treat diseases caused by *Asfarviridae.* It is believed that the viral particles present are eradicated by the MCFA.

In one embodiment, the composition comprises one or more MCFAs selected from the group comprising caproic acid (C6), caprylic acid (C8), pelargonic acid (C9), capric acid (C10), lauric acid (C12) and derivatives thereof. The inventors have found that these MCFAs and derivatives thereof are especially efficient against *Asfarviridae* such as African Swine Fever.

In an embodiment, one MCFA or its derivative thereof is used. Feed supplements comprising caproic acid (C6), caprylic acid (C8), pelargonic acid (C9), capric acid (C10) or lauric acid (C12) and derivatives thereof are given to the animal. In a preferred embodiment, C8 is given to the animal.

In another embodiment, use is made of a composition/feed additive comprising a mixture of specific different MCFAs, the individual MCFAs containing a different number of carbon atoms. Possible mixtures comprise at leastC8, supplemented with one or more MCFAs chosen from caproic acid (C6), pelargonic acid (C9), capric acid (C10) or lauric acid (C12). In another embodiment, mixtures may be based on pelargonic acid (C9), supplemented with one or more MCFAs chosen from caproic acid (C6), caprylic acid (C8), capric acid (C10) or lauric acid (C12). In yet another embodiment, mixtures may be based on C10, supplemented with one or more MCFAs chosen from caproic acid (C6), pelargonic acid (C9), caprylic acid (C8) or lauric acid (C12). Mixtures may also be based on C12, supplemented with one or more MCFAs chosen from caproic acid (C6), pelargonic acid (C9), capric acid (C10) or caprylic acid (C8).

In an embodiment, said composition comprises equal amounts of C8 and C10 and optionally another MCFA chosen from C6-C12. In an embodiment, the concentration of both C8 and C10 in said composition will be between 20 and 50% each of the total amount of said composition.

In an embodiment, said MCFAs preferably comprise:
- between 40% and 100% caprylic acid (C8) or a derivative thereof;
- between 0% and 40% caproic acid (C6) or a derivative thereof;
- between 0% and 40% capric acid (C10) or a derivative thereof;
- between 0% and 20% lauric acid (C12) or a derivative thereof; and
- between 0% and 10% pelargonic acid (C9) or a derivative thereof based on the total weight of the MCFAs.

The inventors have found that a composition comprising a mixture of MCFAs or MCFA derivatives as described above shows optimal preventive and curative properties towards the virus responsible for causing the African Swine Fever disease. In an embodiment, said MCFA fraction comprises at least 40% caprylic acid (C8), more preferably at least 50%, 55%, 60%, 65%, 70%, 75%, 80%, 90%, 95%, 99% of C8 (based on the total weight of said MCFA fraction). According to a further or another embodiment, said MCFAs consist of 100% caprylic acid (C8).

A further or another embodiment of the invention relates to a composition wherein the MCFAs comprise both C8 and C9. In an embodiment, said MCFA fraction of said composition comprises at least 40% C8 and at least 30% C9, more preferably at least 40% C8 and at least 40% C9, more preferably at least 45% C8 and at least 45% C9. In another embodiment, said MCFA fraction in said composition comprises at least 40% C8, and further an amount of C9, such as 1% C9, 5% C9, 10% C9, 15% C9, 20% C9, 25% C9, 30% C9, 35% C9, 40% C9, 45% C9, 50% C9, 55% C9 or 60% C9.. In a more preferred embodiment, said MCFA fraction comprises C8 and C9 in a 1:1 ratio, for instance 50% C8 and 50% C9.

Due to this outstanding effectiveness on prevention, suppression and/or treatment of African Swine Fever, only small doses of the composition according to the invention need to be used to obtain the desired results on animal health and the concomitant positive effects on productivity. Therefore, the current invention leads to higher revenues for the farmers while reducing the costs of disease prevention, treatment and/or suppression.

In a further embodiment, the total amount of MCFAs and/or MCFA derivatives is comprised between 1% and 100% based on the total weight of the composition/feed additive, preferably between 5% and 90%, more preferably between 15% and 80%, even more preferably between 30% and 80%, based on the total weight of the composition. The inventors have found that within the ranges mentioned above, the efficiency of the composition/feed additive for use in the prevention, treatment and / or suppression of African Swine Fever is further increased.

In one embodiment, the concentration of the medium-chain fatty acid, as described herein, amounts at least 1, 5, 10, 15, 20, 25, 30, 35, 40, 45, 50, 55, 60, 65, 70, 75, 80, 85 , 90, 95, 99 % by weight of the composition/feed additive. In a further embodiment, the medium-chain fatty acids, as described herein, amounts to (based on dry weight) between 1 g/100 g composition (1% by weight) and 100 g/100 g composition (100% by weight), preferably between 50 g/100 g and 99 g/ 100 g (50 - 99% by weight), 50 g/ 100 g and 95 g /100 g (50 - 95 % by weight), more preferably 50 g/100 g and 90 g/100 g composition (50-90% by weight), more preferably between 60 g/100g and 80 g/100g. It is to be understood that the concentration of the MCFAs such as described herein maximally amounts to 100% by weight of the composition.

The composition of the invention can be conveniently used in the treatment, suppression and / or prevention of African Swine Fever, as it can be administered orally to the animals.

Oral administration of the composition is attained by feeding the composition to the animals. This method of administration provides the advantage that it is easy to perform and it does not impose the burden of large investments for novel equipment on the farmer.

In a further embodiment, the composition/feed additive further comprises vitamins, minerals, trace elements or a combination thereof. Vitamins include but are not limited to Vitamin A, Vitamin D3, Vitamin E, Vitamin K3, Thiamin, Riboflavin, Panthothenic acid, Biotin, Folic acid, Vitamin B12, Niacin, Pyridoxine, Ascorbic acid, Inositol and Choline. Minerals and trace elements include but are not limited to magnesium, sodium, manganese, iron, zinc, copper, selenium, phosphorous, cobalt and iodine. The addition of these components further contributes to a complete and healthy diet of the animals and positively influences their zootechnical performance. Inclusion of these components in the composition simplifies the steps needed for feeding of the animals as these components no longer need to be added separately to the feed, resulting in an improved efficiency of the feeding process. In addition, the provision of MCFAs and/or MCFA derivatives in the animals' feed was found to result in an increase in feed palatability, which in turn leads to animals that eat more, grow bigger, faster and / or healthier, which eventually leads to an increase in the profitability of the swine production.

In a further embodiment, the composition/feed additive according to the invention as described herein, is formulated as a liquid or as a solid form. The term "solid form" means a powder, a granule or a pellet in particular. The term "liquid form", in particular, means a solution in water or means a solution in oil and includes a viscous paste and a non-viscous solution. The MCFAs and / or MCFA derivatives as described above are oil-soluble and can be provided both as powder and as an oil-solution. In particular, the composition is suitable for oral administration. A feed comprising the composition according to an embodiment of the invention is produced or manufactured by means known to a person skilled in the art. In an embodiment, the feed comprising the composition according to the invention is provided as a dry extruded feed pellet. This formulation allows a relatively long shelf life and also permits the packaging and storage of large amounts of feed.

Oral administration of the composition can be performed by admixing the animal feed with the composition of the invention prior to feeding, by feeding the animal with an animal feed already comprising the composition. Accordingly, the invention provides a feed or feed ingredient comprising a feed additive/ composition for use as described above, wherein the dosage of said composition in said feed or feed ingredient is between 1500 and 5000 ppm by weight, such as 2000 ppm or 3750 ppm based on the total weight of said feed.

Alternatively, said dosage of the MCFA fraction in said feed, feed ingredient or drinking water is between 1500 and 5000 ppm by weight, such as 2000 ppm or 3750 ppm.

According to a further or another embodiment, the dosages as herein described result in treatment of animals with a daily dosage of between 150 and 3000 ppm, preferably between 200 and 2500 ppm, more preferably between 300 and 2000 ppm of MCFAs.

In a further embodiment, the feed further comprises other well-known ingredients so as to provide a nutritionally balanced complete food, including, but not limited to, plant matter, e.g., flour, meal, starch or cracked or processed grain produced from a crop plant such as wheat or other cereals, alfalfa, corn, oats, potato, rice, soybeans or other legumes; cellulose in a form that may be obtained from wood pulp, grasses, plant leaves, and waste plant matter such as rice or soy bean hulls, or corn cobs; animal matter, e.g., fish or crustacean meal, oil, protein or solubles and extracts, krill, meat meal, bone meal, feather meal, blood meal, or cracklings; algal matter; yeast; bacteria; vitamins, minerals, and amino acids; organic binders or adhesives; and chelating agents and preservatives.

Preferably, the animals are treated at least once per day, more preferably two or more times per day such as, for example, 2-6 or 4-6 times per day. It is preferred that any excess food for treatment by oral administration be removed after the feeding period, e.g., by flushing out of a raceway system, or through removal out of the feed trunks.

Oral administration of the composition or MCFAs can also be performed by admixing the animal drinking water with the composition of the invention. Accordingly, the invention provides a drinking water comprising a feed additive/ composition for use as described above, wherein the dosage of said composition or MCFAs in said feed is between 1500 and 5000 ppm by weight, such as 2000 ppm or 3750 ppm based on the total weight of said drinking water.

According to a further or another embodiment, the composition further comprises one or more organic acids chosen from the group of propionic acid, acetic acid, lactic acid, formic acid, citric acid, oxalic acid, malic acid, or combinations thereof. The organic acids comprised herein result in a composition which is soluble in water and therefore is ideally suited for oral administration by admixing the composition with animal drinking water. By preference, said organic acids are present in the composition in a concentration of between 60% and 80% on the total weight of the composition.

The composition according to some embodiments further comprises one or more stabilizers and / or emulsifiers which improve the mixability of the composition in animal drinking water. Such stabilizers may comprise glycerol. By preference, the composition comprises said emulsifiers in a concentration of between 10% and 20% on the total weight of the composition. In a further or another embodiment, the composition comprises said stabilizers in a concentration of between 0,5% and 2,5% on the total weight of the composition.

By preference, the animal is a (domestic) pig, or a warthog.

A further aspect of the present invention relates to a method of inhibiting viruses of the *Asfaraviridae* in animal feed or drinking water, preferably of African Swine Fever virus, said method comprising dosing a composition to said animal feed or drinking water, said composition comprising one or more medium-chain fatty acids (MCFAs) and / or derivatives thereof. The method in accordance with the present invention uses an effective amount of a chemical mitigant to inhibit porcine viruses, preferably ASFV, , in animal feed or drinking water, for example, to concentrations below the levels of detection through cytopathic effect (CPE), RT-PCR and/or virus isolation in cell culture. As used herein, an "effective amount" refers to an amount capable of providing bioavailable levels of the active compound (e.g., medium chain fatty acids) sufficient to achieve the desired performance improvement.

In one or more embodiments, MCFAs for use in the present invention include caproic acid, caprylic acid, capric acid, pelargonic acid and/or lauric acid. Therefore, in certain embodiments, the chemical inhibitor is selected from the group consisting of caproic acid, caprylic acid, capric acid, pelargonic acid, lauric acid, and mixtures thereof.

As discussed above, in one or more embodiments, a blend of medium chain fatty acids may be used. Above-mentioned preferred embodiments are considered to be applicable for the purpose of inhibiting the viruses in animal feed or drinking water. For example, in one or more embodiments, a blend of two or more medium chain fatty acids may be introduced to the feed or drinking water. At least 40% of the total weight of said MCFAs according to the method is caprylic acid (C8). In a preferred embodiment, a 100% C8 MCFA fraction is used.

In a further embodiment, a blend of caprylic acid and pelargonic acid is introduced to the feed or drinking water, for instance at a weight ratio of about 1: 1. In a further preferred embodiment, said the MCFA fraction used comprises 50% C8 and 50% C9. It will be understood that the method as herein described preferably refers to the composition and all of its embodiments described above, and that the effects and advantages thereof equally apply to the present method.

By preference, the composition or MCFA fraction is dosed in said animal feed or drinking water at a dosage of between 1500 and 5000 ppm on the total weight of said animal feed or drinking water, such as 2000 ppm or 3750 ppm based on the total weight of said animal feed or drinking water.

A further aspect of the present invention equally relates to a method of lowering the viral titer of African Swine Fever virus in an animal feed or drinking water, said method comprising adding a composition to said animal feed or drinking water, said composition comprising one or more medium-chain fatty acids (MCFAs) and / or derivatives thereof. It will be understood that the method as herein described preferably refers to the composition and all of its embodiments described above, and that the effects and advantages thereof equally apply to the present method.

In a further embodiment, the method includes the use of MCFAs selected from the group comprising caproic acid (C6), caprylic acid (C8), pelargonic acid (C9), capric acid (C10), lauric acid (C12), derivatives thereof or a combination thereof.

By preference, the method comprises the use of at least caprylic acid (C8) and optionally one or more further MCFAs chosen from caproic acid (C6), pelargonic acid (C9), capric acid (C10), lauric acid (C12), and/or derivatives thereof.

In a further embodiment, the method includes the use of at least 40% of the total weight caprylic acid (C8). According to a further or another embodiment, the method comprises the use of 100% caprylic acid (C8).

In another embodiment, the method further includes the use of pelargonic acid (C9). More preferably, the method includes the use of a blend of caprylic acid and pelargonic acid introduced to the feed or drinking water at a weight ratio of about 1: 1, such as a MCFA fraction comprising 50% C8 and 50% C9.

It is to be understood that the concentration of the MCFAs such as described herein minimally amount to 1% and maximally amounts to 100% by weight or volume of the composition.

The composition or MCFA fraction is dosed between 1500 and 5000 ppm by weight, such as 2000 ppm or 3750 ppm based on the total weight of said animal feed or drinking water.

Embodiments of the present invention advantageously provide a safe alternative method of preventing or decreasing viral infections by the Asfarviridae family, such as African Swine Fever Virus (ASFV), in animal feed or drinking water. Prior methods using harmful chemicals have displayed negative effects on protein and amino acid metabolism of animals. Unlike prior methods, the present invention uses generally non-hazardous chemical mitigants at doses discovered to achieve effective mitigation of viral infections by the Asfarviridae family. The chemical mitigants used in accordance with the present invention are natural alternatives that pose essentially no risk to the safety of workers or the environment.

Additional advantages of the various embodiments of the invention will be apparent to those skilled in the art upon review of the disclosure herein and the working examples below. It will be appreciated that the various embodiments described herein are not necessarily mutually exclusive unless otherwise indicated herein. For example, a feature described or depicted in one embodiment may also be included in other embodiments, but is not necessarily included. Thus, the present invention encompasses a variety of combinations and/or integrations of the specific embodiments described herein.

The invention is further described by the following non-limiting examples which further illustrate the invention, and are not intended to, nor should they be interpreted to, limit the scope of the invention.

### EXAMPLES

### Example 1. The susceptibility to medium chain fatty acid (MCFA) in feed and water spiked with African Swine Fever Virus

### A. INFORMATION CONCERNING MCFA COMPOSITIONS

### 1. Compositions for animal feed application

a. Composition F1:
   100% caprylic acid (C8);
b. Composition F2:
   33% caproic acid (C6), 33% caprylic acid (C8) and 33% capric acid (C10);
c. Composition F3:
   50% caprylic acid (C8), 35% capric acid (C10) and 15% lauric acid (C12);
d. Composition F4:
   95% caprylic acid (C8) and 5% pelargonic acid (C9).

### 2. Compositions for drinking water application

a. Composition W1:
   14% MCFAs, 15% emulsifiers, 70% organic acids and 1% glycerol, MCFAs consisting of 100% caprylic acid (C8);
b. Composition W2:
   14% MCFAs, 15% emulsifiers, 70% organic acids and 1% glycerol, MCFAs consisting of 33% caproic acid (C6), 33% caprylic acid (C8) and 33% capric acid (C10);
c. Composition W3:
   14% MCFAs, 15% emulsifiers, 70% organic acids and 1% glycerol, MCFAs consisting of 50% caprylic acid (C8), 35% capric acid (C10) and 15% lauric acid (C12);
d. Composition W4:
   14% MCFAs, 15% emulsifiers, 70% organic acids and 1% glycerol, MCFAs consisting of 95% caprylic acid (C8) and 5% pelargonic acid (C9).

### B. INFORMATION CONCERNING AFRICAN SWINE FEVER VIRUS (ASFV)

The ASFV strain used for all experiments was isolated in the Red River Delta Region (sample from Hanoi, Vietnam). This strain has been confirmed by virus isolation, HAD assay and real-time PCR as recommended by OIE (the World Organization for Animal Health).

The ASFV strain as used herein has the following genetic characterization, belonging to genotype II, serotype VIII and contain a TRS gene, indicating that it is very close and thus considered representative to ASFV strains detected in China and Belgium, 2018 and differs from ASFV isolated in Georgia in 2007. The Virus Stock Titration used herein equals 10⁷ HAD₅₀/mL (heme adsorbing dose).

### C. EXPERIMENTAL DESIGN

### 1. Animal feed experimental design

### Control groups

Treatment of the feed with animal feed compositions before contamination with ASFV.
- Positive control: complete feed containing 10⁵ HAD₅₀/g of ASFV
- Negative control: complete feed, no ASFV added
- Sampling time-point: after 24 hour exposure
- Incubation of the samples at room temperature (lab condition) for 24 hours
- Evaluated by real-time PCR based on OIE protocol

### Experimental groups

- Feed experiment 1
   Composition F1 treated feed at different doses (1250, 2500, 3750 and 5000 ppm) will be spiked with ASFV and final virus titration is 10⁵ HAD₅₀/g of feed.
- Feed experiment 2
   Composition F2 treated feed at different doses (1250, 2500, 3750 and 5000 ppm) will be spiked with ASFV and final virus titration is 10⁵ HAD₅₀/g of feed.
- Feed experiment 3
   Composition F3 treated feed at different doses (1250, 2500, 3750 and 5000 ppm) will be spiked with ASFV and final virus titration is 10⁵ HAD₅₀/g of feed.
- Feed experiment 4
   Composition F4 treated feed at different doses (1250, 2500, 3750 and 5000 ppm) will be spiked with ASFV and final virus titration is 10⁵ HAD₅₀/g of feed.

### 2. Drinking water experimental design

### Control groups

Treatment of the drinking water with drinking water compositions before contamination with ASFV.
- Positive control: fresh water treated with ASFV isolated in Vietnam at different doses, 10¹ HAD₅₀, 10² HAD₅₀ and 10³ HAD₅₀
- Negative control: fresh water (ASFV negative by real-time PCR)
- Sampling time-point: 30 min, 1, 3, 6, 12 and 24 hours after virus incubation at room temperature
- Evaluated by real-time PCR based on OIE protocol

### Experimental groups

- Water experiment 1
   Composition W1 treated water at different doses (1250, 2500, 3750 and 5000 ppm) will be spiked with ASFV and final virus titration is 10¹ HAD₅₀, 10² HAD₅₀ and 10³ HAD₅₀/mL
- Water experiment 2
   Composition W2 treated water at different doses (1250, 2500, 3750 and 5000 ppm) will be spiked with ASFV and final virus titration is 10¹ HAD₅₀, 10² HAD₅₀ and 10³ HAD₅₀/mL
- Water experiment 3
   Composition W3 treated water at different doses (1250, 2500, 3750 and 5000 ppm) will be spiked with ASFV and final virus titration is 10¹ HAD₅₀, 10² HAD₅₀ and 10³ HAD₅₀/mL
- Water experiment 4
   Composition W4 treated water at different doses (1250, 2500, 3750 and 5000 ppm) will be spiked with ASFV and final virus titration is 10¹ HAD₅₀, 10² HAD₅₀ and 10³ HAD₅₀/mL

### 3. Product treatment

### Animal feed treatment (Kansas State University, 2019)

- Virus working stock of 10⁶ HAD₅₀/ml was used
- To ensure a homogeneous spread of the compositions on the feed, 100g of complete feed was treated with each composition at different doses (1250, 2500, 3750 and 5000 ppm
- Consequently, 22.5g of treated feed was placed in a glass bottle (250 mL) and kept at room temperature for 24h
- 2.5 mL of virus working stock was added to each bottle, obtaining a final virus titration of 10⁵HAD₅₀/g of feed
- The samples were incubated at room temperature for 24 hour
- Sample preparation: 100 mL PBS buffer was placed in each bottle containing 22.5 g of treated feed, 200 µL of supernatant from each sample was used for DNA extraction using QIAamp DNA Mini Kit
- Real-time PCR was performed based on the OIE protocol

### Drinking water treatment

- Virus working stock of 10⁴, 10³ and 10² HAD₅₀/ml was used
- 200 mL of fresh water was treated with each composition at different doses (1250, 2500, 3750 and 5000 ppm)
- Treated water was vortexed and kept at room temperature for 24h
- 22.5 mL of treated water was placed in 50 ml tubes, each tube was spiked with 2.5 mL of 10⁴, 10³ and 10² HAD₅₀ of ASFV, obtaining a final virus titration of 10³ HAD₅₀, 10² HAD₅₀ and 10¹ HAD₅₀/mL of water
- Sample preparation: at time-points 30 min, 1, 3, 6, 12 and 24 hours after virus incubation, samples were collected for DNA extraction and DNA was stored at -80°C until real-time PCR
- Real-time PCR was performed based on the OIE protocol

### 4. DNA preparation

For DNA preparation, a QIAamp DNA Mini Kit was used (Qiagen).

### 5. Real-time PCR

Real-time PCR was employed to evaluate the effect of MCFA on ASFV isolated in Vietnam, based on the OIE protocol "SOP/CISA/ASF/PR/2 Standard operation procedure for the detection of African Swine Fever Virus (ASFV) by real-time polymerase chain reaction (PCR)", 2018. Results of the real-time PCR protocol are expressed in quantification cycles (Cq) required to detect viral nucleic acid. Hence, high Cq values need to be interpreted in the sense that less viral nucleic acid is present.

### 6. Statistical analysis

Statistical analysis was performed using IBM SPSS software (SPSS 23.0 for Windows; IBM, Chicago, IL, USA). A p-value < 0.05 was considered to be statistically significant. Differences among the groups were tested by Duncan's multiple comparison methods.

### D. RESULTS

### 1. Susceptibility of African Swine Fever Virus (ASFV) to medium chain fatty acids (MCFAs) in animal feed

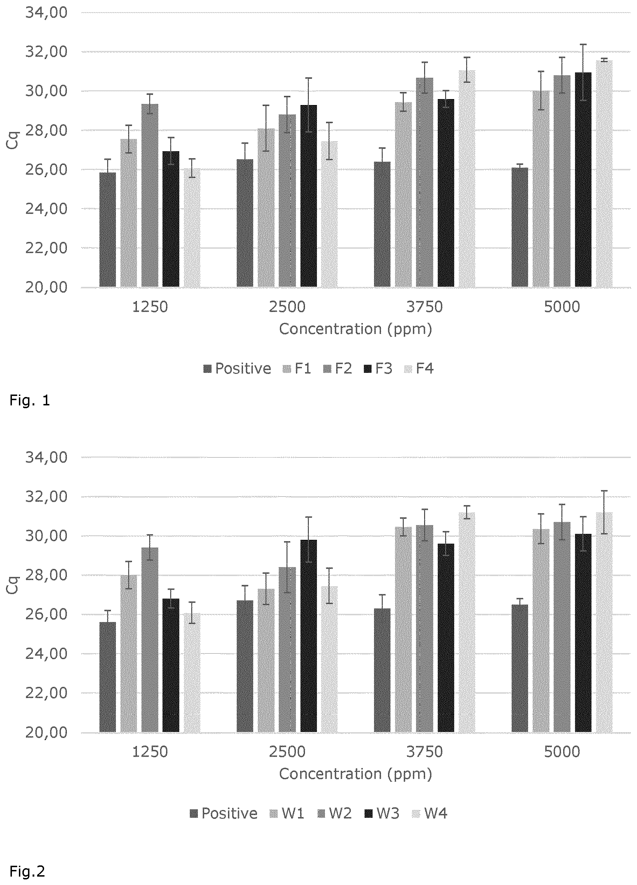

Results of the experiments described above concerning treated animal feed are shown in Figure 1. Results demonstrate that all compositions, including F1, F2, F3 and F4, significantly increase the Cq value for ASFV when compared to the positive control (P<0.01) at highest doses of 3750 and 5000 ppm.

In lower doses, composition F3 with a concentration of 2500 ppm significantly enhanced the Cq value when compared to positive control (P<0.05). Composition F2 shows particularly effective as all doses (1250, 2500, 3750 and 5000 ppm) induced a statistical increase in Cq value when compared to positive control (P<0.05).

From the results as herein discussed, it can be concluded that all tested compositions for feed treatment show significant efficacy for inhibiting/reducing ASFV in animal feed.

### 2. Susceptibility of African Swine Fever Virus (ASFV) to medium chain fatty acids (MCFAs) in drinking water

Results of the experiments described above concerning treated drinking water are shown in Figure 2. Results demonstrate that all compositions, including W1, W2, W3 and W4, significantly increase the Cq value for ASFV when compared to the positive control (P<0.01) at highest doses of 3750 and 5000 ppm.

In lower doses, composition W3 with a concentration of 2500 ppm significantly enhanced the Cq value when compared to positive control (P<0.05). Composition W2 shows particularly effective as all doses (1250, 2500, 3750 and 5000 ppm) induced a statistical increase in Cq value when compared to positive control (P<0.05).

From the results as herein discussed, it can be concluded that all tested compositions for feed treatment show significant efficacy for inhibiting/reducing ASFV in drinking water.

### Example 2. In vitro experiment showing the efficacy of MCFA mixtures

Vero cells were infected with virus particles. The effect on the cells was measured by determining the cytopathic effect (CPE). The CPE was checked using an inverted microscope after 5 days post infection. Different MCFA mixtures were tested at a 0.1% concentration added to the tissue culture. Tests were duplicated. In short, virus suspension was mixed with the MCFA mixtures to be tested and immediately added to a confluent cell monolayer in a 96-well plate (a total volume of 200 µl per well was added). CPE was checked after 5 days of incubation, the results are given in Table 1. The test results for a 100% C12 solution were hampered by the fact that a solubility issue was observed, influencing the results.

The best results were obtained with mixtures containing C8. While the scoring in Table 1 does not allow to distinguish between the various C8 settings, visual scoring allowed to conclude that mixtures with higher C8 concentrations were generally more protective against the cytopathic effect of the virus. Also the mixtures comprising both C8 and C9 scored remarkably well. The 100% C8 mixture and 1:1 C8:C9 scored best.

**Table 1**

| **MCFA mixture added** | **CPE* (first and second test)** |
|---|---|
| 100% C6 | 3/3 |
| 100 % C8 | 2/2 |
| 100 % C9 | 2/2 |
| 100 % C10 | 2/4 |
| 100 % C12 | N.A. |
| 1:1:1 C6/C8/C10 | 3/2 |
| 1:1:1:1 C6/C8/C9/C10 | 2/2 |
| 1:1 C8:C9 | 2/2 |
| Positive control (no virus particles/no MCFA) | 1/1 |
| Negative control (virus particles/no MCFA) | 4/4 |

| | |
|---|---|
| * 4= total destruction of the cells/ 3= medium destruction / 2= low destruction / 1= no destruction | |

## Claims

1. A composition for use in the treatment, suppression and/or prevention of viral infections of the *Asfaraviridae* and the spreading thereof, preferably African Swine Fever virus wherein said composition comprises one or more medium-chain fatty acids (MCFAs) and/or derivatives thereof wherein said MCFAs are chosen from the group comprising fatty acids with a longest continuous chain of 5 carbon atoms to 12 carbon atoms and derivatives thereof **characterized in that** at least 40% of said MCFAs in said composition is caprylic acid (C8) and wherein said MCFAs are dosed in a feed or drinking water at a dosage of between 1500 ppm and 5000 ppm.

2. The composition for use according to claim 1, wherein said MCFAs further comprise pelargonic acid (C9).

3. The composition for use according to any of the previous claims **characterized in that** said MCFAs comprise
between 40% and 100% caprylic acid (C8) or a derivative thereof;
between 0% and 40% caproic acid (C6) or a derivative thereof;
between 0% and 40% capric acid (C10) or a derivative thereof;
between 0% and 20% lauric acid (C12) or a derivative thereof; and
between 0% and 10% pelargonic acid (C9) or a derivative thereof
based on the total weight of the MCFAs.

4. The composition for use according to any of the previous claims, **characterized in that** said MCFAs consist of 100% caprylic acid (C8).

5. The composition for use according to any of the claims 1 to 2, wherein said MCFAs are caprylic acid (C8) and pelargonic acid (C9) in a 1:1 ratio.

6. The composition for use according to any one of the previous claims, **characterized in that** a therapeutically effective amount of said composition is administered orally to said animal, preferably by adding said composition to the feed or drinking water of said animal.

7. The composition for use according to any one of the previous claims, **characterized in that** said composition further comprises one or more organic acids chosen from the group of propionic acid, acetic acid, lactic acid, formic acid, citric acid, oxalic acid, malic acid, or combinations thereof.

8. The composition for use according to any one of the previous claims, **characterized in that** said composition is administered to an animal, said animal is a domestic pig, or a warthog.

9. A method of inhibiting viruses of the *Asfaraviridae* in animal feed or drinking water, preferably of African Swine Fever virus, said method comprising dosing a composition to said animal feed or drinking water, said composition comprising one or more medium-chain fatty acids (MCFAs) and/or derivatives thereof wherein said MCFAs are selected from the group comprising caproic acid (C6), caprylic acid (C8), pelargonic acid (C9), capric acid (C10), lauric acid (C12), derivatives thereof or a combination thereof and wherein at least 40% of the total weight of said MCFAs in said composition is caprylic acid (C8) and wherein said MCFA are dosed to said feed or drinking water at a dosage of between 1500 ppm and 5000 ppm.

10. The method according to claim 9, wherein said MCFAs further comprise pelargonic acid (C9).

11. The method according to claims 9 or 10 **characterized in that** said MCFAs comprise
- between 40% and 100% caprylic acid (C8) or a derivative thereof;
- between 0% and 40% caproic acid (C6) or a derivative thereof;
- between 0% and 40% capric acid (C10) or a derivative thereof;
- between 0% and 20% lauric acid (C12) or a derivative thereof; and
- between 0% and 10% pelargonic acid (C9) or a derivative thereof based on the total weight of the MCFAs.

12. A method of lowering the viral titer of African Swine Fever virus in an animal feed or drinking water, said method comprising adding a composition to said animal feed or drinking water, said composition comprising one or more medium-chain fatty acids (MCFAs) and/or derivatives thereof wherein said MCFAs are selected from the group comprising caproic acid (C6), caprylic acid (C8), pelargonic acid (C9), capric acid (C10), lauric acid (C12), derivatives thereof or a combination thereof and wherein at least 40% of the total weight of said MCFAs in said composition is caprylic acid (C8) and wherein said MCFA are dosed to said feed or drinking water at a dosage of between 1500 ppm and 5000 ppm.

13. The method according to claim 12, wherein said MCFAs further comprise pelargonic acid (C9).

14. The method according to claims 12 or 13, **characterized in that** said MCFAs comprise
- between 40% and 100% caprylic acid (C8) or a derivative thereof;
- between 0% and 40% caproic acid (C6) or a derivative thereof;
- between 0% and 40% capric acid (C10) or a derivative thereof;
- between 0% and 20% lauric acid (C12) or a derivative thereof; and
- between 0% and 10% pelargonic acid (C9) or a derivative thereof based on the total weight of the MCFAs.

## Patentansprüche

1. Zusammensetzung zur Verwendung bei der Behandlung, Unterdrückung und/oder Prävention viraler Infektionen mit *Asfaraviridae* und deren Verbreitung, vorzugsweise das Virus der Afrikanischen Schweinepest, wobei die Zusammensetzung eine oder mehrere mittelkettige Fettsäuren (MCFAs) und/oder Derivate davon umfasst, wobei die MCFAs aus der Gruppe ausgewählt sind, die Fettsäuren mit einer längsten kontinuierlichen Kette von 5 Kohlenstoffatomen bis 12 Kohlenstoffatomen und Derivate davon umfasst, **dadurch gekennzeichnet, dass** mindestens 40 % der MCFAs in der Zusammensetzung Caprylsäure (C8) sind und wobei die MCFAs mit einer Dosierung zwischen 1500 ppm und 5000 ppm in ein Futter oder Trinkwasser dosiert werden.

2. Zusammensetzung zur Verwendung nach Anspruch 1, wobei die MCFAs ferner Pelargonsäure (C9) umfassen.

3. Zusammensetzung zur Verwendung nach einem der vorhergehenden Ansprüche, **dadurch gekennzeichnet, dass** die MCFAs, bezogen auf das Gesamtgewicht der MCFAs, Folgendes umfassen:
zwischen 40 % und 100 % Caprylsäure (C8) oder ein Derivat davon,
zwischen 0 % und 40 % Capronsäure (C6) oder ein Derivat davon,
zwischen 0 % und 40 % Caprinsäure (C10) oder ein Derivat davon,
zwischen 0 % und 20 % Laurinsäure (C12) oder ein Derivat davon und
zwischen 0 % und 10 % Pelargonsäure (C9) oder ein Derivat davon.

4. Zusammensetzung zur Verwendung nach einem der vorhergehenden Ansprüche, **dadurch gekennzeichnet, dass** die MCFAs aus 100 % Caprylsäure (C8) bestehen.

5. Zusammensetzung zur Verwendung nach einem der Ansprüche 1 bis 2, wobei die MCFAs Caprylsäure (C8) und Pelargonsäure (C9) in einem Verhältnis von 1:1 sind.

6. Zusammensetzung zur Verwendung nach einem der vorhergehenden Ansprüche, **dadurch gekennzeichnet, dass** eine therapeutisch wirksame Menge der Zusammensetzung dem Tier oral verabreicht wird, vorzugsweise durch Zusetzen der Zusammensetzung zum Futter oder Trinkwasser des Tiers.

7. Zusammensetzung zur Verwendung nach einem der vorhergehenden Ansprüche, **dadurch gekennzeichnet, dass** die Zusammensetzung ferner eine oder mehrere organische Säuren umfasst, die aus der Gruppe der Propionsäure, der Essigsäure, der Milchsäure, der Ameisensäure, der Zitronensäure, der Oxalsäure, der Äpfelsäure oder Kombinationen daraus ausgewählt ist.

8. Zusammensetzung zur Verwendung nach einem der vorhergehenden Ansprüche, **dadurch gekennzeichnet, dass** die Zusammensetzung einem Tier verabreicht wird, wobei das Tier ein Hausschwein oder ein Warzenschwein ist.

9. Verfahren zum Hemmen von Viren der *Asfaraviridae* in Tierfutter oder Trinkwasser, vorzugsweise des Virus der Afrikanischen Schweinepest, wobei das Verfahren das Dosieren einer Zusammensetzung in Tierfutter oder -trinkwasser umfasst, wobei die Zusammensetzung eine oder mehrere mittelkettige Fettsäuren (MCFAs) und/oder Derivate davon umfasst, wobei die MCFAs aus der Gruppe ausgewählt sind, die Capronsäure (C6), Caprylsäure (C8), Pelargonsäure (C9), Caprinsäure (C10), Laurinsäure (C12), Derivate davon oder eine Kombination daraus umfasst und wobei mindestens 40 % des Gesamtgewichts der MCFAs in der Zusammensetzung Caprylsäure (C8) sind und die MCFAs mit einer Dosierung zwischen 1500 ppm und 5000 ppm in das Futter oder Trinkwasser dosiert werden.

10. Verfahren nach Anspruch 9, wobei die MCFAs ferner Pelargonsäure (C9) umfassen.

11. Verfahren nach Anspruch 9 oder 10, wobei die MCFAs, bezogen auf das Gesamtgewicht der MCFAs, Folgendes umfassen:
zwischen 40 % und 100 % Caprylsäure (C8) oder ein Derivat davon,
zwischen 0 % und 40 % Capronsäure (C6) oder ein Derivat davon,
zwischen 0 % und 40 % Caprinsäure (C10) oder ein Derivat davon,
zwischen 0 % und 20 % Laurinsäure (C12) oder ein Derivat davon und
zwischen 0 % und 10 % Pelargonsäure (C9) oder ein Derivat davon.

12. Verfahren zum Senken des Virustiters des Virus der Afrikanischen Schweinepest in einem Tierfutter oder -trinkwasser, wobei das Verfahren das Zusetzen einer Zusammensetzung zu dem Tierfutter oder -trinkwasser umfasst, wobei die Zusammensetzung eine oder mehrere mittelkettige Fettsäuren (MCFAs) und/oder Derivate davon umfasst, wobei die MCFAs aus der Gruppe ausgewählt werden, die Capronsäure (C6), Caprylsäure (C8), Pelargonsäure (C9), Caprinsäure (C10), Laurinsäure (C12), Derivate davon oder eine Kombination daraus umfasst, und wobei mindestens 40 % des Gesamtgewichts der MCFAs in der Zusammensetzung Caprylsäure (C8) sind und wobei die MCFAs mit einer Dosierung zwischen 1500 ppm und 5000 ppm in das Futter oder Trinkwasser dosiert werden.

13. Verfahren nach Anspruch 12, wobei die MCFAs ferner Pelargonsäure (C9) umfassen.

14. Verfahren nach Anspruch 12 oder 13, **dadurch gekennzeichnet, dass** die MCFAs, bezogen auf das Gesamtgewicht der MCFAs, Folgendes umfassen:
zwischen 40 % und 100 % Caprylsäure (C8) oder ein Derivat davon,
zwischen 0 % und 40 % Capronsäure (C6) oder ein Derivat davon,
zwischen 0 % und 40 % Caprinsäure (C10) oder ein Derivat davon,
zwischen 0 % und 20 % Laurinsäure (C12) oder ein Derivat davon und
zwischen 0 % und 10 % Pelargonsäure (C9) oder ein Derivat davon.

## Revendications

1. Composition destinée à être utilisée dans le traitement, la suppression et/ou la prévention des infections virales des *Asfaraviridae* et de leur propagation, de préférence le virus de la peste porcine africaine, dans laquelle ladite composition comprend un ou plusieurs acides gras à chaîne moyenne (AGCM) et/ou leurs dérivés, dans laquelle lesdits AGCM sont choisis dans le groupe comprenant les acides gras avec une chaîne continue la plus longue de 5 atomes de carbone à 12 atomes de carbone et leurs dérivés, **caractérisée en ce qu'**au moins 40 % desdits AGCM dans ladite composition est de l'acide caprylique (C8), et dans laquelle lesdits AGCM sont dosés dans un aliment ou une eau de boisson à un dosage compris entre 1500 ppm et 5000 ppm.

2. Composition destinée à être utilisée selon la revendication 1, dans laquelle lesdits AGCM comprennent en outre de l'acide pélargonique (C9).

3. Composition destinée à être utilisée selon l'une quelconque des revendications précédentes, **caractérisée en ce que** lesdits AGCM comprennent
entre 40 % et 100 % d'acide caprylique (C8) ou d'un dérivé de celui-ci ;
entre 0 % et 40 % d'acide caproïque (C6) ou d'un dérivé de celui-ci ;
entre 0 % et 40 % d'acide caprique (C10) ou d'un dérivé de celui-ci ;
entre 0 % et 20 % d'acide laurique (C12) ou d'un dérivé de celui-ci ; et
entre 0 % et 10 % d'acide pélargonique (C9) ou d'un dérivé de celui-ci
par rapport au poids total des AGCM.

4. Composition destinée à être utilisée selon l'une quelconque des revendications précédentes, **caractérisée en ce que** lesdits AGCM sont constitués à 100 % d'acide caprylique (C8).

5. Composition destinée à être utilisée selon l'une quelconque des revendications 1 à 2, dans laquelle lesdits AGCM sont l'acide caprylique (C8) et l'acide pélargonique (C9) dans un rapport 1:1.

6. Composition destinée à être utilisée selon l'une quelconque des revendications précédentes, **caractérisée en ce qu'**une quantité thérapeutiquement efficace de ladite composition est administrée par voie orale audit animal, de préférence en ajoutant ladite composition à l'aliment ou à l'eau de boisson dudit animal.

7. Composition destinée à être utilisée selon l'une quelconque des revendications précédentes, **caractérisée en ce que** ladite composition comprend en outre un ou plusieurs acides organiques choisis dans le groupe constitué par l'acide propionique, l'acide acétique, l'acide lactique, l'acide formique, l'acide citrique, l'acide oxalique, l'acide malique, ou des combinaisons de ceux-ci.

8. Composition destinée à être utilisée selon l'une quelconque des revendications précédentes, **caractérisée en ce que** ladite composition est administrée à un animal, ledit animal est un porc domestique ou un phacochère.

9. Procédé d'inhibition des virus des *Asfaraviridae* dans les aliments ou l'eau de boisson pour animaux, de préférence du virus de la peste porcine africaine, ledit procédé comprenant le dosage d'une composition dans ledit aliment ou ladite eau de boisson pour animaux, ladite composition comprenant un ou plusieurs acides gras à chaîne moyenne (AGCM) et/ou leurs dérivés, dans lequel lesdits AGCM sont choisis dans le groupe comprenant l'acide caproïque (C6), l'acide caprylique (C8), l'acide pélargonique (C9), l'acide caprique (C10), l'acide laurique (C12), leurs dérivés ou une combinaison de ceux-ci, et dans lequel au moins 40 % du poids total desdits AGCM dans ladite composition est de l'acide caprylique (C8) et dans lequel lesdits AGCM sont dosés dans ledit aliment ou ladite eau de boisson à un dosage compris entre 1500 ppm et 5000 ppm.

10. Procédé selon la revendication 9, dans lequel lesdits AGCM comprennent en outre de l'acide pélargonique (C9).

11. Procédé selon les revendications 9 ou 10, **caractérisé en ce que** lesdits AGCM comprennent
- entre 40 % et 100 % d'acide caprylique (C8) ou d'un dérivé de celui-ci ;
- entre 0 % et 40 % d'acide caproïque (C6) ou d'un dérivé de celui-ci ;
- entre 0 % et 40 % d'acide caprique (C10) ou d'un dérivé de celui-ci ;
- entre 0 % et 20 % d'acide laurique (C12) ou d'un dérivé de celui-ci ; et
- entre 0 % et 10 % d'acide pélargonique (C9) ou d'un dérivé de celui-ci par rapport au poids total des AGCM.

12. Procédé de réduction du titre viral du virus de la peste porcine africaine dans un aliment ou une eau de boisson pour animaux, ledit procédé comprenant l'ajout d'une composition audit aliment ou à ladite eau de boisson pour animaux, ladite composition comprenant un ou plusieurs acides gras à chaîne moyenne (AGCM) et/ou des dérivés de ceux-ci, dans lequel lesdits AGCM sont choisis dans le groupe comprenant l'acide caproïque (C6), l'acide caprylique (C8), l'acide pélargonique (C9), l'acide caprique (C10), l'acide laurique (C12), des dérivés de ceux-ci ou une combinaison de ceux-ci, et dans lequel au moins 40 % du poids total desdits AGCM dans ladite composition est de l'acide caprylique (C8), et dans lequel lesdits AGCM sont dosés dans ledit aliment ou ladite eau de boisson à un dosage compris entre 1500 ppm et 5000 ppm.

13. Procédé selon la revendication 12, dans lequel lesdits AGCM comprennent en outre de l'acide pélargonique (C9).

14. Procédé selon les revendications 12 ou 13, **caractérisé en ce que** lesdits AGCM comprennent
- entre 40 % et 100 % d'acide caprylique (C8) ou d'un dérivé de celui-ci ;
- entre 0 % et 40 % d'acide caproïque (C6) ou d'un dérivé de celui-ci ;
- entre 0 % et 40 % d'acide caprique (C10) ou d'un dérivé de celui-ci ;
- entre 0 % et 20 % d'acide laurique (C12) ou d'un dérivé de celui-ci ; et
- entre 0 % et 10 % d'acide pélargonique (C9) ou d'un dérivé de celui-ci par rapport au poids total des AGCM.
